# EUROPEAN PATENT APPLICATION

(11) **EP 1 121 933 A1**
(43) Date of publication of application: **08.08.2001**
(21) Application number: 00300827.3
(22) Date of filing: 02.02.2000
(51) Int. Cl.: A61K 31/4375, A61K 47/02, A61K 47/10, A61K 47/26, A61K 9/08

(54) **Premixed alatrofloxacin injectable compositions**

(71) Applicant: Pfizer Products Inc., Groton, Connecticut 06340 (US)
(72) Inventor: Harper, Nancy Jane, Groton, Connecticut 06340 (US); Ranade, Gautam Ramchandra, Groton, Connecticut 06340 (US); Qi, Hong, Northbrook, Illinois 60062 (US)
(74) Representative: Simpson, Alison Elizabeth Fraser

(57) **Abstract**

The present invention provides premixed (ready-to-use) pharmaceutical compositions for intravenous administration containing an aqueous solution of alatrofloxacin or a pharmaceutically acceptable salt thereof and pharmaceutically acceptable tonicity agents. The present invention also provides methods of using such compositions to treat or prevent a variety of bacterial infections.

## Description

### FIELD OF THE INVENTION

The present invention provides sterile, premixed (ready-to-use) pharmaceutical compositions for intravenous administration comprising an aqueous solution of alatrofloxacin or a pharmaceutically acceptable salt thereof and pharmaceutically acceptable tonicity agents. The present invention also provides methods of using said compositions to treat or prevent a variety of bacterial infections.

### BACKGROUND OF THE INVENTION

Trovafloxacin is a fluorinated quinolone antibacterial of formula I

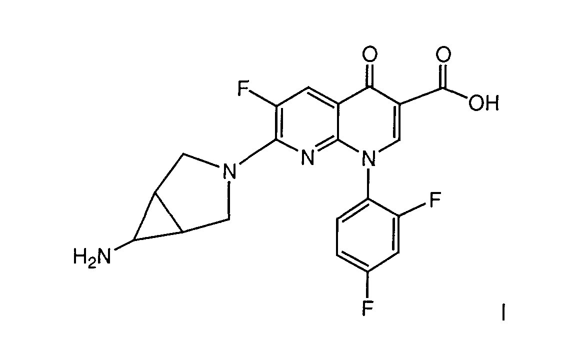

It has the following chemical name: (1α, 5α, 6α)-7-(6-amino-3-azabicyclo [3.1.0] hex-3-yl)-1-(2,4-difluorophenyl)-6-fluoro-1,4-dihydro-4-oxo-1,8-naphthyridine-3-carboxylic acid. Trovafloxacin and its salts, such as the mesylate salt, are disclosed in U.S. Patent No. 5,164,402, which issued on 17 November 1992, and which is incorporated herein by reference.

Alatrofloxacin is a prodrug of trovafloxacin and is the compound of formula II

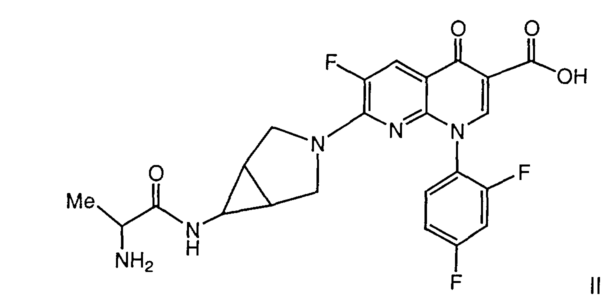

It has the following chemical name: (1α, 5α, 6α)-L-alanyl-N-[3-[6-carboxy-8-(2,4-difluorophenyl)-3-fluoro-5,8-dihydro-5-oxo-1,8-naphthyridin-2-yl]-3-azabicyclo [3.1.0] hex-6-yl]-L-alaninamide. The antibacterial activity and synthesis of alatrofloxacin are described in the U.S. Patent No. 5,164,402, which issued on 17 November 1992, and U.S. Patent No. 5,229,396, which issued on 20 July 1993, the disclosures of which are incorporated by reference herein.

International Patent Publication WO 97/00268, which published on 3 January 1997, discloses an alternative process for preparing alatrofloxacin. The foregoing publication is hereby incorporated by reference herein.

International Patent Publication WO 97/08191, which published on 6 March 1997, describes a third process for the preparation of alatrofloxacin, as well as polymorphs thereof, the disclosure of which is hereby incorporated by reference herein.

International Patent Publication WO 95/19361, which published on 20 July 1995; Published European patent application 0818445, published 14 January 1998; and Published European patent application 0863874, published 16 September 1998; discloses novel intermediates for preparing alatrofloxacin and trovafloxacin, the disclosures of which are hereby incorporated by reference herein.

U.S. Patent No. 5,475,116, which issued on 12 December 1995, and U.S. Patent No. 5,256,791, which issued on 26 October 1993, disclose various azabicyclohexane intermediates useful in the synthesis of alatrofloxacin and trovafloxacin, the disclosures of which are hereby incorporated by reference herein. U.S. Patent No. 5,391,763, which issued on 21 February 1995, discloses 3-aza-bicyclco[3.1.0]hexanes which are intermediates for antibacterial azabicyclo quinolone carboxylic acids, the disclosure of which is hereby incorporated by reference herein.

U.S. Patent No. 5,266,569, which issued on 30 November 1993, discloses azatricyclo carboxylic acids which are useful as anti-bacterial agents, the disclosure of which is hereby incorporated by reference herein.

U.S. Patent No. 5,728,711, which issued on 17 March 1998, discloses the use of compounds, such as trovafloxacin and alatrofloxacin, to treat *H. pylori* infections and to treat gastric and duodenal ulcers. The foregoing patent is hereby incorporated by reference herein.

These patents disclose dosages and modes of administration for these antibacterial compounds. In particular, these patents disclose that the compounds can be injected parenterally, for example, intramuscularly, intravenously or subcutaneously. They also state that for parenteral administration, these compounds are best used in the form of a sterile aqueous solution which can contain other solutes, for example, enough salt or glucose to make the solution isotonic.

International Patent Application, PCT/IB98/01122, filed 23 July 1998 (published as WO 99/06430 on 11 February 1999), and which designates, *inter alia,* the United States, relates to alatrofloxacin mesylate essentially free of less polar impurities and parenteral compositions thereof, and to processes for purifying alatrofloxacin mesylate. According to this application, for parenteral administration (intramuscular, intraperitoneal, subcutaneous and intravenous use) a sterile pyrogen free injectable solution of the active ingredient is usually prepared. That application discloses that solutions of alatrofloxacin mesylate, substantially free of less polar impurities, can be prepared in a solvent such as sesame oil, peanut oil, water or aqueous propylene glycol. It discloses that preferably the solution is unbuffered water and that the aqueous solutions can also be suitably adjusted and buffered, if necessary, and the liquid diluent first rendered isotonic. It discloses that such aqueous solutions are suitable for intravenous injection purposes and that the oily solutions are suitable for intraarticular, intramuscular and subcutaneous injection purposes. It also discloses the current commercial intravenous formulation of alatrofloxacin mesylate, which is described further below, and describes specific parenteral compositions of purified alatrofloxacin mesylate which contain 500 mg of dextrose.

U.S. Patent No. 5,763,454, which issued on 9 June 1998, discloses a novel crystal form of anhydrous trovafloxacin mesylate, a method of using the compound in the treatment of a bacterial infection in mammals, especially humans, and pharmaceutical compositions of the compound, the disclosure of which is hereby incorporated by reference herein.

International Patent Publication WO 97/07800, which published on 6 March 1997, claims zwitterionic forms of trovafloxacin, which are useful for the administration of the compound as a suspension, the disclosure of which is hereby incorporated by reference herein.

Trovafloxacin is available as TROVAN™ Tablets (trovafloxacin mesylate) for oral administration and as TROVAN™ IV (alatrofloxacin mesylate injection), a prodrug of trovafloxacin, as noted above, for intravenous administration. (Physicians' Desk Reference (PDR), 52nd ed., Supplement A, Medical Economics Co., Inc., Montvale, NJ, pages A 72- A79 (1998).)

TROVAN™ Tablets contain trovafloxacin mesylate, a synthetic broad-spectrum antibacterial agent for oral administration. The tablets are available in 100 mg and 200 mg (trovafloxacin equivalent) blue, film-coated tablets.

TROVAN IV™ contains alatrofloxacin mesylate, the L-alanyl-L-alanyl prodrug of trovafloxacin mesylate. It is intended for intravenous administration following dilution in a suitable IV diluent. Following intravenous administration, the alanine substituents in alatrofloxacin are rapidly hydrolyzed *in vivo* to yield trovafloxacin. TROVAN™ IV is available in 40 ml and 60 ml single use vials as a sterile, preservative-free aqueous concentrate of 5 mg trovafloxacin/ml as alatrofloxacin mesylate intended for dilution prior to intravenous administration of doses of 200 mg or 300 mg of trovafloxacin, respectively. The formulation contains water for injection, and may contain sodium hydroxide or hydrochloric acid for pH adjustment. The pH range for the 5 mg/ml aqueous concentrate is 3.5 to 4.3. After intravenous administration, alatrofloxacin is rapidly converted to trovafloxacin.

The intravenous dose can be prepared by aseptically withdrawing the approriate volume of concentrate from the vials of TROVAN™ IV. This can then be diluted with a suitable intravenous solution to a final concentration of 1-2 mg/ml. The resulting solution is infused over a period of sixty minutes by direct infusion or through a Y-type intravenous infusion set, which may already be in place. The following are indicated as being compatible intravenous solutions: 5% dextrose; 0.45% sodium chloride; 5% dextrose and 0.45% sodium chloride; 5% dextrose and 0.2% sodium chloride; Lactated Ringer's injection and 5% dextrose. (Physicians' Desk Reference (PDR), 52nd ed., Supplement A, Medical Economics Co., Inc., Montvale, NJ, pages A 72- A79 (1998).)

Parenteral drug products containing glycerin are known in the art and are commercially available. For example, glucagon for injection, which is a lyophile formulation for treating hypoglycemia, contains 1.6% glycerin; and D.H.E. 45® Injection (dihydroergotamine mesylate) which is a solution formulation for treating migraine, contains 15% glycerin. (Physicians' Desk Reference (PDR), 52nd ed., Medical Economics Co., Inc., Montvale, NJ, pages 1455-1458; and pages1840-1842 (1998).) DIPRIVAN® 1% Injectable Emulsion, which is a sterile, nonpyrogenic emulsion for use as an IV sedative-hypnotic agent, contains glycerol (22.5 mg/ml). (Physicians' Desk Reference (PDR), 52nd ed., Medical Economics Co., Inc., Montvale, NJ, pages 3157-3163 (1998).)

However, IV drug products containing glycerin are conventionally for nutritional use. For example, ProcalAmine, which is an IV solution for nutrition, contains 3% glycerin; Liposyn II 10% and 20%, which are IV emulsions for nutrition, contains 2.5% glycerin; and Intralipid injection 10% and 20%, which are IV emulsions for nutrition, contains 2.25% glycerin. Drug Facts and Comparisons, pages 36a, 36g and 40 to 40b, Facts and Comparisons, Inc., Wolters Kluwer Co., St. Louis, Mo. (August 1996).

Published European patent application 0 394 026, published 24 October 1990, discloses aqueous pharmaceutical formulations containing the antiviral drugs, 1-isobutyl-1H-imidazo (4,5-c) quinolin-4-amine or 1-(2-hydroxy-2-methyl-propyl)-1H-imidazo (4,5-c) quinolin-4-amine, which formulations are suitable for parenteral administration. Those formulations contains those compounds, which are dissolved in an aqueous acid, and a tonicity adjuster, such as glycerin, sorbitol and dextrose.

J. Pharm. Sci. Technol. (1994), 48(2), 86-91, discloses the development of a parenteral liquid formulation of a renin inhibitor compound in which 2.5% mannitol is the tonicity modifier.

Eur. J. Cancer 34, Suppl. 2, S27, 1998, discloses a ready-to-use (RTU) formulation of idarubicin HCL for intravenous injection which contains glycerol.

As described above, the currently marketed IV formulation of alatrofloxacin requires transfer of the product to another container and mixing with a diluent prior to administration. These manipulations prior to use increase the personnel time and risk of contamination. Therefore, there is a need in the art for an alternative IV dosage form of alatrofloxacin that is premixed and ready-to-use.

### SUMMARY OF THE INVENTION

The present invention particularly provides:

A pharmaceutical composition for intravenous administration which comprises:
a sterile, premixed aqueous solution comprising an antibacterially effective amount of alatrofloxacin, or a pharmaceutically acceptable salt thereof, and two or more tonicity agents;
wherein one of the tonicity agents is sodium chloride, which is present at a concentration of not more than about 0.45%; and
wherein the other tonicity agent or agents are selected from the group consisting of dextrose, sucrose, mannitol, glycerin, sorbitol and glycine; provided that if dextrose is present, it is present at a concentration which is less than about 5%.

The composition of the present invention preferably contains tonicity agents which are sodium chloride and one or more of the following: sucrose, mannitol, glycerin, sorbitol and glycine. Most preferably, this composition contains tonicity agents which are sodium chloride, which is present at a concentration of not more than about 0.23%, and glycerin.

More particularly, in one embodiment, the composition of the present invention comprises alatrofloxacin mesylate, sodium chloride and glycerin. Even more particularly, the composition has a solution pH of about 3 to about 5.

Optionally, the composition of the present invention further comprises a buffering agent. Also, optionally, the composition of the present invention further comprises one or more pharmaceutically acceptable preservatives.

Most particularly, in one embodiment, the composition of the present invention comprises alatrofloxacin mesylate, sodium chloride, glycerin, lactic acid and a solution pH of about 3 to about 5.

Finally, the present invention provides method of treating or preventing bacterial infections which comprises administering the composition of the present invention to a patient in need thereof.

### DETAILED DESCRIPTION OF THE INVENTION

The compositions of the present invention are new dosage forms of alatrofloxacin, which are ready-to-use aqueous solutions for direct IV infusion without further dilution. Advantageously, these compositions do not require any manipulations to the dosage form prior to use, thereby minimizing dose preparation time and reducing possible sources of contamination.

The compositions of the present invention comprise the active ingredient, alatrofloxacin, or a pharmaceutically acceptable salt thereof, such as the mesylate salt or the hydrochloride salt, at about 0.5 mg to about 7.0 mg/ml. Most preferably, the compositions comprise alatrofloxacin mesylate, and it is most preferably present at about 3.14 mg/ml. This amount of alatrofloxacin mesylate is equivalent to a drug concentration of about 2 mg/ml of trovafloxacin, which is the parent, non-salt form of the drug. However, the actual quantity of alatrofloxacin mesylate needed to be equivalent to a drug concentration of about 2 mg trovafloxacin per ml may be adjusted based on the actual potency of the drug substance lot used.

In addition to the active ingredient, the compositions of the present invention contain tonicity agents (which may also be referred to as osmolarity or osmolality adjusting agents) which agents make the compositions compatible with the circulatory system. An isotonic or iso-osmotic solution is one that has the same or substantially the same osmotic pressure as blood. The isotonicity or iso-osmolarity of a solution is readily determined by one of ordinary skill in the pharmaceutical arts.

Preferred compositions of the present invention are approximately iso-osmotic or isotonic. Such compositions are optimally tolerated by the patient. Some of the compositions of the present invention are hypotonic, but are still acceptable for intravenous administration according to the present invention.

However, development of a ready-to-use IV dosage form of alatrofloxacin has been complicated by the incompatibility of alatrofloxacin with traditional tonicity agents, such as sodium chloride (also known as salt or saline) or dextrose (also known as glucose). Typically sodium chloride or dextrose is used as a tonicity agent in IV products. However, alatrofloxacin at its preferred concentrations is physically incompatible with isotonic or iso-osmotic concentrations of sodium chloride, because it results in the precipitation of the hydrochloride salt of alatrofloxacin. Also, alatrofloxacin demonstrates chemical incompatibility in the presence of isotonic concentrations of dextrose on long-term storage. Therefore, they would not be preferred as single tonicity agents for use in a ready-to-use IV product of alatrofloxacin.

However, surprisingly, it has been found that tonicity agents in combination with lower concentrations of sodium chloride (which is a preferred tonicity agent) may be used in the compositions of the present invention. In the composition of the present invention, a concentration of not more than about 0.45% sodium chloride should be present. A variety of other tonicity agents is available in the pharmaceutical arts. These agents include, for example, dextrose, sucrose, mannitol, glycerin, sorbitol and glycine. In the composition of the present invention, if dextrose is present as a tonicity agent, it should be present at a hypotonic concentration (which is less than about 5%).

The binary tonicity agents, sodium chloride (also known as salt or saline) and glycerin (also known as glycerine or glycerol), are preferred. The sodium chloride is present at about 0.1 to about 4.5 mg/ml. Most preferably, it is present at about 2.3 mg/ml. The glycerin is present at about 0.1 to about 28 mg/ml. Most preferably, it is present at about 18.6 mg/ml. With binary tonicity agents, one of ordinary skill in the art would be able to adjust the amount of each agent in order to maintain the physiologically appropriate osmolarity or osmolality of the composition. Likewise, with ternary tonicity agents, one of ordinary skill in the art would be able to make a similar adjustment to the amount of each such agent.

Optionally, the compositions of the present invention also contain ingredients to adjust the pH of the solution. For example, sodium hydroxide and/or hydrochloric acid may be used in the compositions of the present invention for pH adjustment. A final pH range of about 3.0 to about 5.0 provides the optimal chemical and physical stability of the solution of the present invention. More preferably, the pH of the solution is about 3.0 to about 4.0. Most preferably, the pH of the solution is about 3.5.

Also, optionally, the compositions of the present invention contain a buffering agent, which maintains the pH of the composition. Examples of suitable buffering agents include acetic acid, citric acid and, preferably, lactic acid. The lactic acid is present at about 0.1 to about 2 mg/ml. Most preferably, it is present at about 0.34 mg/ml.

Optionally, a nitrogen headspace in the finished compositions is present to improve the product's oxidative stability and therefore prolong the product's shelflife.

The compositions of the present invention also optionally contain one or more pharmaceutically acceptable preservatives, which preserve the quality of the compositions from a chemical or microbiological standpoint. Preservatives, such as antioxidants, metal chelators, metal complexing agents and antimicrobial agents, may be used in the compositions of the present invention. These preservatives may be used in the compositions, both alone and in various combinations (e.g., antioxidant(s) in combination with a metal complexing agent), to improve the stability of the compositions.

Examples of preservatives, which may be present in the compositions of the present invention, include the following: propyl gallate, ascorbic acid, sodium metabisulfite, sodium bisulfite, ethylenediamine tetraacetic acid (EDTA) or its salts (such as the disodium salt), citric acid and phenol. Also, various combinations of these preservatives may be present in the compositions of the present invention, such as propyl gallate and disodium EDTA.

The amount of preservative that may be present in the compositions of the present invention ranges from about 0.01 to about 0.5 mg/ml of the composition; most preferably, preservatives, such as propyl gallate and disodium EDTA, are present in an amount of about 0.2 mg/ml of the composition.

As described above, the compositions of the present invention are ready-to-use products which offer convenience of administration. No further dilution of these formulations is necessary. The daily doses for alatrofloxacin mesylate range from about 100 to about 300 mg as trovafloxacin; more typically, the daily doses are 200 and 300 mg as trovafloxacin, which can be delivered directly by, for example, a 100 ml or a 150 ml bag, respectively. This product should be administered by intravenous infusion over a period of about 60 minutes.

The manufacture of the compositions of the present invention utilizes conventional pharmaceutical equipment and processes. For example, in one embodiment, compounding involves dissolving the excipients and the drug in nitrogen-sparged water for injection, then adjusting the pH as necessary with 1 N NaOH in water for injection (WFI) and/or 1 N HCI in WFI. The solution is filtered through a prefilter and other final filters, and then filled into containers, such as flexible bags or glass bottles.

For example, the bags are sealed and inserted into a foil overwrap. A nitrogen headspace is introduced into the overwrap, which is then immediately sealed. The bags are terminally moist heat sterilized. To allow administration of label claim of 200 mg and 300 mg (as trovafloxacin), minimum overfills of 2 ml and 3 ml may be included in the respective presentations. These quantities represent 2% overfill for the 100 ml and 150 ml bags, respectively. Alternatively, the compositions of the present invention can be aseptically manufactured, subjected to sterile filtration and then filled into containers, thus avoiding the heat stress of terminal sterilization.

### EXAMPLES

### EXAMPLE 1

Ready-to-Use Formulation (2 mg Trovafloxacin /ml) with Binary Tonicity agents at pH 3.50

| Ingredient | g/1000 ml | mg/1000 ml | mg/ml | % (w/v) (g/100ml) | mg/100 ml | mg/150 ml |
|---|---|---|---|---|---|---|
| | | | | | | |
| Alatrofloxacin Mesylate | 3.14 | 3140 | 3.14 | 0.31 | 314 | 471 |
| | | | | | | |
| Sodium Chloride | 2.30 | 2300 | 2.3 | 0.23 | 230 | 345 |
| | | | | | | |
| Glycerin | 18.60 | 18600 | 18.60 | 1.86 | 1860 | 2790 |
| | | | | | | |
| Lactic Acid | 0.34 | 341 | 0.34 | 0.03 | 34 | 51 |
| | | | | | | |
| Water for Injection | 979.32 | 979320 | 979.32 | 97.93 | 97932 | 146898 |
| | | | | | | |
| Total | 1003.7 | 1003701 | 1003.7 | 100.37 | 100370 | 150555 |
| | | | | | | |

Sodium Chloride, Glycerin, Lactic Acid, and Drug were dissolved in Water For Injection, and were titrated to pH 3.5 with 5% NaOH solution.

A solution as shown in the table above containing 3.14 mg/ml alatrofloxacin mesylate was prepared in 1.86% glycerin, 0.23% sodium chloride and 0.034% lactic acid solution, with pH adjusted to 3.5 (+ or - 0.5) by sodium hydroxide (about 0.027 to about 0.037 g/1000 ml; on average about 0.032 g/1000ml). The above composition stored in either flexible containers or glass vials has demonstrated acceptable physical and chemical stability over at least 12 months when stored at room temperature (15°C to 30°C) and protected from light. No significant increase in particulate or haze was observed under the same conditions.

### EXAMPLE 2

One ml of an aqueous solution containing:
3.14 Alatrofloxacin mesylate;
2.3 mg sodium chloride;
12.5 mg dextrose;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 2A

One ml of an aqueous solution containing:
3.14 Alatrofloxacin mesylate;
2.3 mg sodium chloride;
33.3 mg dextrose;
0.9 mg lactic acid;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 3

One ml of an aqueous solution containing:
3.14 mg alatrofloxacin mesylate;
2.3 mg sodium chloride;
25 mg sucrose;
hydrochloric acid or sodium hydroxide solution to 3.75;
water-for-injection: sufficient quantity to produce 1 ml of solution;
Alatrofloxacin mesylate and excipients were dissolved in water-for-injection with stirring.

### EXAMPLE 3A

One ml of an aqueous solution containing:
3.14 mg alatrofloxacin mesylate;
2.3 mg sodium chloride;
66.6 mg sucrose;
0.9 mg lactic acid;
hydrochloric acid or sodium hydroxide solution to 3.00;
water-for-injection: sufficient quantity to produce 1 ml of solution;
Alatrofloxacin mesylate and excipients were dissolved in water-for-injection with stirring.

### EXAMPLE 3B

One ml of an aqueous solution containing:
3.14 mg alatrofloxacin mesylate;
3.0 mg sodium chloride;
50.0 mg sucrose;
0.9 mg lactic acid;
hydrochloric acid or sodium hydroxide solution to 3.75;
water-for-injection: sufficient quantity to produce 1 ml of solution;
Alatrofloxacin mesylate and excipients were dissolved in water-for-injection with stirring.

### EXAMPLE 4

One ml of an aqueous solution containing:
3.14 mg alatrofloxacin mesylate;
2.3 mg sodium chloride;
12.5 mg mannitol;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 4A

One ml of an aqueous solution containing:
3.14 mg alatrofloxacin mesylate;
2.3 mg sodium chloride;
33.3 mg mannitol;
hydrochloric acid or sodium hydroxide solution to pH 3.4;
water-for-injection: sufficient quantity to produce 1 ml of solution;
Alatrofloxacin mesylate and excipients were dissolved in water-for-injection with stirring.

### EXAMPLE 4B

One ml of an aqueous solution containing:
3.14 mg alatrofloxacin mesylate;
2.3 mg sodium chloride;
33.3 mg mannitol;
0.9 mg lactic acid;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 5

One ml of an aqueous solution containing:
3.14 mg alatrofloxacin mesylate;
2.3 mg sodium chloride;
6.0 mg glycerin;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 5A

One ml of an aqueous solution containing:
3.14 mg alatrofloxacin mesylate;
2.3 mg sodium chloride;
18.6 mg glycerin;
0.9 mg lactic acid;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 6

One ml of an aqueous solution containing:
3.14 mg alatrofloxacin mesylate;
2.3 mg sodium chloride;
12.5 mg sorbitol;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 6A

One ml of an aqueous solution containing:
3.14 mg alatrofloxacin mesylate;
2.3 mg sodium chloride;
33.3 mg sorbitol;
0.9 mg lactic acid;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 7

One ml of an aqueous solution containing:
3.14 mg alatrofloxacin mesylate;
2.3 mg sodium chloride;
15 mg glycine;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 8

One ml of an aqueous solution containing:
3.14 mg alatrofloxacin mesylate;
2.3 mg sodium chloride;
16.7 mg dextrose;
33.3 mg sucrose;
0.9 mg lactic acid;
hydrochloric acid or sodium hydroxide solution to pH 3.75;
water-for-injection: Sufficient Quantity to produce 1 ml of solution;
Alatrofloxacin mesylate and excipients were dissolved in water-for-injection with stirring.

### EXAMPLE 9

One ml of an aqueous solution containing:
3.14 mg alatrofloxacin mesylate;
2.3 mg sodium chloride;
16.7 mg dextrose;
16.7 mg mannitol;
hydrochloric acid or sodium hydroxide solution to pH 3.4;
water-for-injection: sufficient quantity to produce 1 ml of solution;
Alatrofloxacin mesylate and excipients were dissolved in water-for-injection with stirring.

### EXAMPLE 10

One ml of an aqueous solution containing:
3.14 mg alatrofloxacin mesylate;
2.3 mg sodium chloride;
16.7 mg dextrose;
9.4 mg glycerin;
0.9 mg lactic acid;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 10A

One ml of an aqueous solution containing:
3.14 mg alatrofloxacin mesylate;
2.3 mg sodium chloride;
16.7 mg dextrose;
9.3 mg glycerin;
hydrochloric acid or sodium hydroxide solution to pH 3.4;
water-for-injection: sufficient quantity to produce 1 ml of solution;
Alatrofloxacin mesylate and excipients were dissolved in water-for-injection with stirring.

### EXAMPLE 11

One ml of an aqueous solution containing:
3.14 mg alatrofloxacin mesylate;
2.3 mg sodium chloride;
16.7 mg dextrose;
16.7 mg sorbitol;
0.9 mg lactic acid;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 12

One ml of an aqueous solution containing:
3.14 mg alatrofloxacin mesylate;
2.3 mg sodium chloride;
16.7 mg dextrose;
7.5 mg glycine;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 13

One ml of an aqueous solution containing:
3.14 mg alatrofloxacin mesylate;
2.3 mg sodium chloride;
33.3 mg sucrose;
9.4 mg glycerin;
0.9 mg lactic acid;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 14

One ml of an aqueous solution containing:
3.14 mg alatrofloxacin mesylate;
2.3 mg sodium chloride;
33.3 mg sucrose;
7.5 mg glycine;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 15

One ml of an aqueous solution containing:
3.14 mg alatrofloxacin mesylate;
2.3 mg sodium chloride;
16.7 mg mannitol;
9.4 mg glycerin;
hydrochloric acid or sodium hydroxide solution to pH 3.4;
water-for-injection: sufficient quantity to produce 1 ml of solution;
Alatrofloxacin mesylate and excipients were dissolved in water-for-injection with stirring.

### EXAMPLE 16

One ml of an aqueous solution containing:
3.14 mg alatrofloxacin mesylate;
2.3 mg sodium chloride;
9.4 mg glycerin;
7.5 mg glycine;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 17

One ml of an aqueous solution containing:
3.14 mg alatrofloxacin mesylate;
2.3 mg sodium chloride;
9.4 mg glycerin;
16.7 mg sorbitol;
0.9 mg lactic acid;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 18

One ml of an aqueous solution containing:
3.14 mg alatrofloxacin mesylate;
2.3 mg sodium chloride;
16.7 mg sorbitol;
7.5 mg glycine;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 19

One ml of an aqueous solution containing:
3.14 mg alatrofloxacin mesylate;
2.3 mg sodium chloride;
33.3 mg mannitol;
0.9 mg lactic acid;
0.1 mg propyl gallate;
0.1 mg disodium EDTA
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 20

One ml of an aqueous solution containing:
3.14 mg alatrofloxacin mesylate;
2.3 mg sodium chloride;
18.6 mg glycerin;
0.9 mg lactic acid;
0.1 mg propyl gallate;
0.1 mg disodium EDTA;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

### EXAMPLE 21

One ml of an aqueous solution containing:
3.14 mg alatrofloxacin mesylate;
2.3 mg sodium chloride;
33.3 mg sorbitol;
0.9 mg lactic acid;
0.1 mg propyl gallate;
0.1 mg disodium EDTA;
hydrochloric acid or sodium hydroxide solution to pH 3.75;
water-for-injection: sufficient quantity to produce 1 ml of solution;
Alatrofloxacin mesylate and excipients were dissolved in water-for-injection with stirring.

It should be understood that various changes and modifications to the presently preferred embodiments described herein will be apparent to those skilled in the art. Such changes and modifications can be made without departing from the spirit and scope of the present invention and without diminishing its advantages. It is therefore intended that such changes and modifications are within the scope of the appended claims.

## Claims

1. A pharmaceutical composition for intravenous administration which comprises:
a sterile, premixed aqueous solution comprising an antibacterially effective amount of alatrofloxacin, or a pharmaceutically acceptable salt thereof, and two or more tonicity agents;
wherein one of the tonicity agents is sodium chloride, which is present at a concentration of not more than about 0.45%; and
wherein the other tonicity agent or agents are selected from the group consisting of dextrose, sucrose, mannitol, glycerin, sorbitol and glycine; provided that if dextrose is present, it is present at a concentration which is less than about 5%.

2. The composition of claim 1 wherein the pharmaceutically acceptable salt of alatrofloxacin is the mesylate salt.

3. The composition of claim 1 wherein the tonicity agents are sodium chloride and one or more of the following: sucrose, mannitol, glycerin, sorbitol and glycine.

4. The composition of claim 3 wherein the tonicity agents are sodium chloride, which is present at a concentration of not more than about 0.23%, and glycerin.

5. The composition of claim 1 wherein the pharmaceutically acceptable salt of alatrofloxacin is alatrofloxacin mesylate, the tonicity agents are sodium chloride and glycerin, and the pH of the solution is about 3 to about 5.

6. The composition of claim 5 which further comprises a buffering agent selected from the group consisting of citric acid, acetic acid and lactic acid.

7. The composition of claim 6 wherein the alatrofloxacin mesylate is present at about 0.5 to about 7.0 mg/ml, wherein the sodium chloride is present at about 0.1 to about 4.5 mg/ml, wherein the glycerin is present at about 0.1 to about 28 mg/ml, wherein the lactic acid is present at about 0.1 to about 2 mg/ml and wherein the pH of the solution is about 3 to about 5.

8. The composition of claim 7 wherein each milliliter of the composition contains about 3.14 mg of alatrofloxacin mesylate, about 2.3 mg of sodium chloride, about 18.6 mg of glycerin, about 0.34 mg of lactic acid, a solution pH of about 3.5, and a sufficient quantity of water to produce one milliliter.

9. The composition of claim 7 which further comprises one or more pharmaceutically acceptable preservatives selected from the group consisting of propyl gallate, ascorbic acid, sodium metabisulfite, sodium bisulfite, ethylenediamine tetraacetic acid (EDTA) or salts thereof, citric acid and phenol.

10. Use of a composition of claim 1 for the manufacture of a medicament for the treatment or prevention of bacterial infections in a patient in need thereof.
